# FASCICULE DE BREVET EUROPEEN

(11) **EP 3 672 067 B1**
(45) Date de publication et mention de la délivrance du brevet: **28.07.2021**
(21) Numéro de dépôt: 19217035.5
(22) Date de dépôt: 17.12.2019
(51) Int. Cl.: H02S 50/10, G01N 27/02, G01N 33/44

(54) **PROCÉDÉ DE CARACTÉRISATION DU TAUX MOYEN DE RÉTICULATION D'UNE COUCHE D'ENCAPSULATION, PRÉSENTE DANS UN MODULE PHOTOVOLTAÏQUE**
VERFAHREN ZUR CHARAKTERISIERUNG DER DURCHSCHNITTLICHEN VERNETZUNGSRATE EINER IN EINEM PHOTOVOLTAISCHEN MODUL VORGESEHENEN ENCAPSULATION-SCHICHT
METHOD FOR CHARACTERIZING THE AVERAGE CROSSLINKING RATE OF AN ENCAPSULATION LAYER IN A PHOTOVOLTAIC MODULE

(30) Priorité: 20.12.2018 FR 1873667; 20.12.2018 FR 1873661
(43) Date de publication de la demande: 24.06.2020
(73) Titulaire: Commissariat à l'énergie atomique et aux énergies alternatives, 75015 Paris (FR); Université de Lorraine, 54052 Nancy Cedex (FR)
(72) Inventeur: OGIER, Stéphane, 73000 CHAMBERY (FR); COMMAULT, Benjamin, 38054 GRENOBLE cedex 09 (FR); HIDALGO, Manuel, 69530 BRIGNAIS (FR); VITE, Marion, 38054 GRENOBLE Cedex 09 (FR)
(74) Mandataire: Brevalex

(56) Documents cités:
- DE-A1-102009 026 062
- GB-A- 2 158 255

## Description

### DOMAINE TECHNIQUE

L'invention se rapporte à un procédé de caractérisation, non destructif, du taux moyen de réticulation d'une couche d'encapsulation, présente dans un module photovoltaïque. L'invention concerne plus particulièrement les modules photovoltaïques comportant des cellules photovoltaïques de type silicium.

L'invention se rapporte également à un procédé de suivi de la qualité d'un module photovoltaïque. Les documents DE 10 2009 026062 A1 et GB 2 158 255 A décrivent des procédés de caractérisation des dispositifs photovoltaïques.

### ÉTAT DE LA TECHNIQUE ANTÉRIEURE

Le procédé de fabrication d'un module photovoltaïque consiste, classiquement, à empiler, dans un premier temps, les différentes couches constitutives du module :
- une plaque transparente en face avant du module ; la face avant étant celle exposée au rayonnement solaire incident lors de l'implantation des modules sur le terrain,
- une première couche de matériau polymère transparent (premier film encapsulant),
- un ensemble de cellules photovoltaïques interconnectées,
- une deuxième couche de matériau polymère (deuxième film encapsulant),
- une plaque en face arrière du module.

Les films encapsulants, aussi appelés feuilles ou couches d'encapsulation, sont en général positionnés de part et d'autre des cellules photovoltaïques. Les films encapsulants sont des films solides en un matériau polymère de type élastomère, par exemple, en polyoléfines fonctionnelles réticulables, comme le copolymère d'éthylène et d'acétate de vinyle (EVA).

Après l'étape d'assemblage, l'ensemble ainsi formé est soumis à une étape de lamination (aussi appelée étape de laminage), permettant de faire fondre puis réticuler les matériaux polymères des films encapsulants. Cette opération est généralement exécutée à l'aide d'un laminateur (aussi appelé laminoir) pouvant être, par exemple, une presse à membrane. Cette opération peut également être réalisée par un autre procédé, par autoclave par exemple. On obtient ainsi des cellules photovoltaïques complètement encapsulées dans une couche d'encapsulation, obtenue à partir des deux films encapsulants. Les cellules sont isolées de l'environnement extérieur et protégées des chocs et des contraintes mécaniques. L'EVA est un matériau thermofusible, mais grâce à la réticulation intervenant lors du procédé de lamination, il devient réticulé. La lamination thermique de l'empilement a également pour conséquence le collage de l'ensemble de la structure.

L'alternative au procédé de lamination thermique décrit ci-dessus est celle de l'utilisation d'encapsulants sous forme initiale liquide polymérisable qui, pendant l'opération de mise en module photovoltaïque sont capables de se transformer par polymérisation voire réticulation en un matériau solide caoutchoutique souple, protégeant les cellules et assurant le collage ou la cohésion de l'assemblage.

Il est, par exemple, possible d'utiliser des résines liquides à l'état initial, les résines liquides pouvant être solidifiées avec un apport de chaleur, ou sous rayonnement ultra-violet, comme les résines de type silicone ou acrylique.

Dans les deux cas, il est indispensable que les matériaux polymères ou les résines formant les couches d'encapsulation soient suffisamment polymérisés et réticulés pour assurer leur fonction d'encapsulation.

Un faible taux polymérisation/réticulation (aussi appelé taux de conversion) peut mener, par exemple, à des problèmes liés à la présence de monomères ou oligomères non convertis (interaction chimique avec la cellule, dissolution des monomères...), au fluage du matériau polymère et donc à une diminution de la durée de vie du module photovoltaïque.

Par exemple, Pern et al. ont montré qu'une réticulation de l'EVA d'au moins 80% permet d'obtenir de bonnes propriétés thermomécaniques et optiques, et une résistance chimique satisfaisante (« EVA encapsulants for PV modules: Reliability issues and current R&D status at NREL », Renewable Energy, vol 8, 1-4, p.367, 1996).

Pour une résine, un taux de réticulation d'au moins 95% permet d'obtenir de bonnes propriétés thermomécaniques et optiques, et une résistance chimique (propriété barrière) satisfaisante.

Or, le taux de réticulation peut varier en fonction des conditions opérationnelles utilisées (température, pression, durée de l'irradiation ou du chauffage, laminateur, durée de l'étape de laminage, etc) ainsi qu'en fonction du matériau (quantité et nature des agents de réticulation). Il est donc essentiel de pouvoir déterminer le taux de réticulation des couches d'encapsulation obtenues soit par lamination de films encapsulants soit par polymérisation/réticulation d'une résine.

Il existe plusieurs méthodes connues permettant d'assurer un suivi de la réaction de réticulation/conversion d'un polymère.

La première méthode est une méthode par extraction de solvant (Soxhlet). Cette méthode est par exemple décrite dans la norme IEC 62788-1-6 (« Procédures de mesure des matériaux utilisés dans les modules photovoltaïques - Partie 1-6 : Encapsulants - Méthodes d'essai pour déterminer le degré de durcissement dans l'éthylène-acétate de vinyle »). Des échantillons de polymère sont prélevés, immergés dans du xylène et suivent des cycles dans le Soxhlet à 140°C, pendant au moins 8h, puis les résidus sont séchés à 115°C pendant au moins 4h. La fraction insoluble est ensuite déterminée.

Cependant, cette méthode est destructive, particulièrement longue à mettre en œuvre, et utilise des solvants pouvant être toxiques.

Une autre méthode est une méthode par chromatographie gazeuse. Cette méthode a, par exemple, été utilisée par Xiang et al. (« Preparation, Characterization and Application of UV-Curable Flexible Hyperbranched Polyurethane Acrylate », Polymers 2017, 9, 552; doi:10.3390/polym9110552). Dans cet article, la masse moléculaire moyenne en poids et la polydispersité d'un polyuréthane acrylate est déterminée par chromatographie, avec des solvants de type THF.

Il est également possible de déterminer le taux de réticulation par analyse calorimétrique différentielle (ou DSC pour « Differential Scanning Calorimetry »), par spectroscopie Raman ou encore par analyse diélectrique (DEA pour « Dielectric Analysis »). Hardis et al. ("Cure kinetics characterization and monitoring of an epoxy resin using DSC, Raman spectroscopy, and DEA", Compos. Part Appl. Sci. Manuf. 49 (2013) 100-108) ont utilisé ces techniques pour déterminer le taux de réticulation d'une résine époxyde. Pour déterminer le taux de réticulation par DSC, les échantillons réticulés de résine époxyde sont, dans un premier temps, refroidis à -50°C puis chauffés jusqu'à 275°C, à 10°C/min. La température de transition vitreuse est mesurée. Cette température dépend du degré de réticulation. Pour déterminer le taux de réticulation par DEA, une tension de 1V est appliquée à un échantillon de résine de 0,5mm d'épaisseur, positionné entre deux électrodes. La permittivité et le facteur de perte sont mesurés. Le facteur de perte, qui dépend de la mobilité des ions au sein du matériau, diminue avec le degré de réticulation. Concernant la spectroscopie Raman, l'intensité du pic correspondant au groupe époxyde diminue quand le degré de réticulation augmente. Il a été observé que les taux de réticulation déterminés par les trois techniques concordent.

Cependant, les méthodes existantes sont destructives (nécessité de récupérer un morceau de film encapsulant pour l'analyser), chronophages et/ou coûteuses.

### EXPOSÉ DE L'INVENTION

Un but de la présente invention est de proposer un procédé de caractérisation du taux moyen de réticulation d'une couche d'encapsulation, présente dans un module photovoltaïque, sans les inconvénients des méthodes précitées, et notamment sans avoir besoin de prélever et de détruire un échantillon de la couche d'encapsulation.

Pour cela, la présente invention propose un procédé de caractérisation du taux moyen de réticulation d'une couche d'encapsulation, présente dans un module photovoltaïque, comprenant les étapes successives suivantes :
a) fournir un module photovoltaïque comprenant au moins :
   - une première plaque,
   - une pluralité de cellules photovoltaïques, reliées électriquement entre elles,
   - une couche d'encapsulation, encapsulant la pluralité de cellules photovoltaïques,
   - une deuxième plaque, la couche d'encapsulation et la pluralité de cellules photovoltaïques étant situées entre les première et deuxième plaques,
b) recouvrir la première plaque par un élément électriquement conducteur,
c) mesurer la résistance électrique de la couche d'encapsulation entre la pluralité de cellules photovoltaïques et l'élément électriquement conducteur, avec un appareil de mesure,
d) comparer la résistance électrique mesurée avec des valeurs de référence prédéfinies, de manière à déterminer le taux moyen de réticulation de la couche d'encapsulation.

Selon une première variante, la couche d'encapsulation est une couche solide en matériau polymère au moins partiellement réticulée, par exemple, obtenue par lamination d'un ou plusieurs films encapsulants en matériau polymère réticulable. L'étape de lamination permet de faire fondre les films encapsulants et de les faire au moins partiellement réticuler.

Selon cette première variante, par au moins partiellement réticulé, on entend un taux de réticulation supérieur à 50%, et de préférence supérieur à 75%. La réticulation correspond à la formation de liaisons covalentes entre les chaînes polymères macromoléculaires (ponts de réticulation) créant un réseau tridimensionnel.

Selon une deuxième variante, la couche d'encapsulation est une couche solide obtenue à partir d'une résine liquide, qui a été au moins partiellement polymérisée et réticulée, par exemple par apport de chaleur et/ou de rayonnements UV. Une résine qui est polymérisée et réticulée est aussi appelée 'résine convertie'.

Selon cette deuxième variante, par au moins partiellement polymérisée et réticulée (i.e. par au moins partiellement convertie), on entend un taux de réticulation supérieur à 80%, et de préférence supérieur à 90%. La polymérisation correspond à la formation de chaînes macromoléculaires à partir de monomères, oligomères et/ou pré-polymères, et la réticulation correspond à une formation de liaisons covalentes entre les chaînes macromoléculaires (ponts de réticulation) créant un réseau tridimensionnel. Les deux phénomènes se produisent de manière combinée.

Le procédé de l'invention consiste à mesurer la résistance électrique de la couche d'encapsulation, entre le circuit de cellules photovoltaïques et un contact métallique, positionné à l'extérieur du module photovoltaïque (sur la face avant ou sur la face arrière). La résistance du matériau polymère ou de la résine de la couche d'encapsulation reflète la mobilité des porteurs de charges, qui dépend de la mobilité des chaines macromoléculaires et dépend donc de l'avancement de la réaction de polymérisation/réticulation. Cette mesure diélectrique permet de déterminer le taux de réticulation (aussi appelé taux de conversion, degré de polymérisation/réticulation ou avancement de la polymérisation/réticulation) de la couche d'encapsulation.

La mesure est fiable et très rapide (de quelques secondes à quelques minutes).

Le procédé est facile à mettre en œuvre et non destructif. Il permet de contrôler directement la réticulation des modules PV sur la ligne de production (« in line »), ou à la fin de la ligne de production (post-fabrication) ou encore d'analyser des modules PV sur le terrain ou revenant du terrain, ce qui n'est pas possible avec les procédés de l'art antérieur. Le procédé selon l'invention permet d'évaluer le taux de réticulation d'un matériau polymère sur un module photovoltaïque terminé.

Avantageusement, le procédé comporte, en outre, après l'étape d), les étapes successives suivantes :
e) retirer l'élément électriquement conducteur de la première plaque et recouvrir la deuxième plaque avec l'élément électriquement conducteur,
f) répéter étapes c) et d) telles que définies précédemment.

Lorsque la couche d'encapsulation est obtenue en laminant deux films encapsulants, disposés de part et d'autre de la pluralité de cellules photovoltaïques, le procédé peut être réalisé sur la face avant et/ou arrière d'un module photovoltaïque. Il peut permettre de vérifier que le taux moyen de réticulation est suffisant en face avant et/ou en face arrière.

Avantageusement, le procédé comporte entre l'étape b) et l'étape c) les étapes suivantes :
b1) connecter la pluralité de cellules photovoltaïques et l'élément électriquement conducteur, d'une part, à une source de tension ou de courant, et d'autre part, à l'appareil de mesure,
b2) appliquer une tension ou un courant entre la pluralité de cellules photovoltaïques et l'élément électriquement conducteur.

Avantageusement, la tension appliquée lors de l'étape b2) est supérieure ou égale à 250V, par exemple 500V ou 1000V, pour avoir une résistance mesurable avec une précision suffisante.

Avantageusement, le courant ou la tension appliquée lors de l'étape b2) est appliquée pendant une durée d'au moins 30 secondes, et de préférence d'au moins 1 minute. Ceci permet d'atteindre un régime stationnaire.

Avantageusement, l'étape b2) est répétée deux fois, par exemple une première fois à une tension de 1000V et une deuxième fois à une tension de 500V, pour éviter de perturber les mesures avec la charge des éléments capacitifs du module photovoltaïque.

Avantageusement, une pression d'au moins 10Pa est appliquée sur l'élément électriquement conducteur, lors des étapes b2) et c). Le contact entre l'élément électriquement conducteur et la plaque est amélioré.

Avantageusement, le procédé comporte entre l'étape c) et l'étape d) les étapes suivantes :
c1) débrancher l'appareil de mesure, et
c2) décharger le module photovoltaïque en court-circuitant des parties électriquement conductrices du module photovoltaïque avec la terre.

Selon un mode de réalisation avantageux, la couche d'encapsulation est une couche de résine acrylique.

Selon un autre mode de réalisation avantageux, la couche d'encapsulation est une couche de résine silicone.

Selon un autre mode de réalisation avantageux, la couche d'encapsulation comprend et est, avantageusement, constituée par un matériau polymère de type élastomère, par exemple, une polyoléfine fonctionnelle, comme le copolymère d'éthylène et d'acétate de vinyle (EVA) ou un autre copolymère à base d'éthylène.

Selon une variante de réalisation avantageuse, l'élément électriquement conducteur recouvre la pluralité de cellules photovoltaïques. Il est ainsi possible de mesurer la résistance de la partie de la couche encapsulante disposée entre les cellules PV et la plaque recouverte par l'élément électriquement conducteur. On choisira avantageusement un élément électriquement conducteur ayant la même surface que les cellules reliées au générateur de tension/courant et on les disposera en regard l'un de l'autre.

Selon cette variante de réalisation avantageuse, l'élément électriquement conducteur peut être une feuille métallique, par exemple une feuille d'aluminium ou de cuivre.

Selon une autre variante de réalisation avantageuse, l'élément électriquement conducteur est un cadre disposé sur le pourtour du module photovoltaïque.

L'invention concerne également un procédé de suivi qualité d'une ligne de fabrication de modules photovoltaïques, comprenant les étapes successives suivantes :
i. Assembler au moins :
   - une première plaque,
   - un premier film d'encapsulation réticulable,
   - une pluralité de cellules photovoltaïques, reliées électriquement entre elles,
   - un deuxième film d'encapsulation réticulable,
   - une deuxième plaque.
ii. Laminer l'ensemble obtenu à l'étape i, de manière à faire fondre et au moins partiellement réticuler le premier film d'encapsulation réticulable et le deuxième film d'encapsulation réticulable, moyennant quoi on forme un module photovoltaïque ayant une couche d'encapsulation encapsulant la pluralité de cellules photovoltaïques,
iii. Réaliser le procédé de caractérisation du taux moyen de réticulation de la couche d'encapsulation, telles que définies précédemment, pour suivre la qualité du module photovoltaïque.

Avantageusement, si le taux moyen de réticulation de la couche d'encapsulation est inférieur à une valeur seuil prédéfinie, au moins un des paramètres de la ligne de fabrication du module photovoltaïque, par exemple, choisi parmi la température, la pression et la durée de l'étape de lamination, est modifié.

L'invention concerne également un procédé de suivi qualité d'une ligne de fabrication de modules photovoltaïques, comprenant les étapes successives suivantes :
i'. assembler au moins :
   - une première plaque,
   - une couche de formulation liquide polymérisable/réticulable,
   - une pluralité de cellules photovoltaïques,
   - une deuxième plaque.
ii'. Faire polymériser/réticuler, la formulation liquide polymérisable/réticulable, moyennant quoi on forme un module photovoltaïque ayant une couche d'encapsulation encapsulant la pluralité de cellules photovoltaïques,
iii'. Réaliser le procédé de caractérisation du taux moyen de conversion de la couche d'encapsulation, telles que définies précédemment, pour suivre la qualité du module photovoltaïque.

Avantageusement, si le taux moyen de réticulation de la couche d'encapsulation est inférieur à une valeur seuil prédéfinie, au moins un des paramètres de la ligne de fabrication du module photovoltaïque, par exemple, choisi parmi la température, la pression et la durée de l'étape de polymérisation/réticulation, est modifié.

### BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :
La figure la représente, de manière schématique, en vue éclatée, les différents éléments d'un module photovoltaïque, avant une étape de lamination, selon un mode de réalisation particulier de l'invention.
La figure 1b représente, de manière schématique, en vue éclatée, les différents éléments d'un module photovoltaïque, après une étape de lamination, selon un mode de réalisation particulier de l'invention.
La figure 2 représente, de manière schématique, en vue éclatée, un module photovoltaïque, dont la couche d'encapsulation est obtenue à partir d'une résine liquide, selon un mode de réalisation particulier de l'invention.
La figure 3 représente de manière schématique, le montage électrique pour mesurer la résistance d'une couche d'encapsulation d'un module photovoltaïque, selon un mode de réalisation particulier de l'invention.
La figure 4 représente la résistance d'une couche d'EVA en fonction du taux moyen de réticulation.
La figure 5 représente l'évolution de la conductivité d'une formulation acrylique photopolymérisable en fonction du temps sans irradiation UV et sous rayonnement UV.
La figure 6 représente le taux de conversion mesuré par variation de permittivité en fonction du taux de conversion mesuré par spectroscopie Raman d'une résine époxyde.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

### EXPOSÉ DÉTAILLÉ DE MODES DE RÉALISATION PARTICULIERS

### Assemblage et lamination du module photovoltaïque

On se réfère tout d'abord à la figure la qui représente une vue éclatée de différents éléments que l'on assemble pour fabriquer un module photovoltaïque (PV), selon un mode de réalisation particulier de l'invention. Le module photovoltaïque est obtenu en assemblant :
- une première plaque 10, en face avant du module,
- une première couche de matériau polymère 21 (premier film encapsulant),
- un ensemble de cellules photovoltaïques 30, par exemple, en silicium, disposées côte à côte, et interconnectées entre elles, par des connections métalliques 31 électriquement conductrices,
- une deuxième couche de matériau polymère 22 (deuxième film encapsulant),
- une deuxième plaque 40, en face arrière.

Puis l'ensemble ainsi formé est laminé à chaud.

Initialement, c'est-à-dire avant l'opération de lamination, le module comprend, avantageusement, au moins deux films encapsulants 21, 22, entre lesquels la pluralité de cellules photovoltaïques 30 est disposée. Selon une alternative, non représentée, le module pourrait comprendre un seul film d'encapsulation. Selon une autre variante non représentée, le module pourrait comprendre plusieurs films encapsulants disposés en face avant et/ou plusieurs films encapsulants disposés en face arrière.

Les films encapsulants 21, 22 utilisés, lors de l'assemblage, sont des films en matériau polymère réticulable, c'est-à-dire qu'ils sont polymérisés mais non réticulés ou peu réticulés.

Par non réticulés ou peu réticulés, on entend qu'ils ont de préférence un taux de réticulation inférieur ou égal à 5% et de préférence inférieur à 1%.

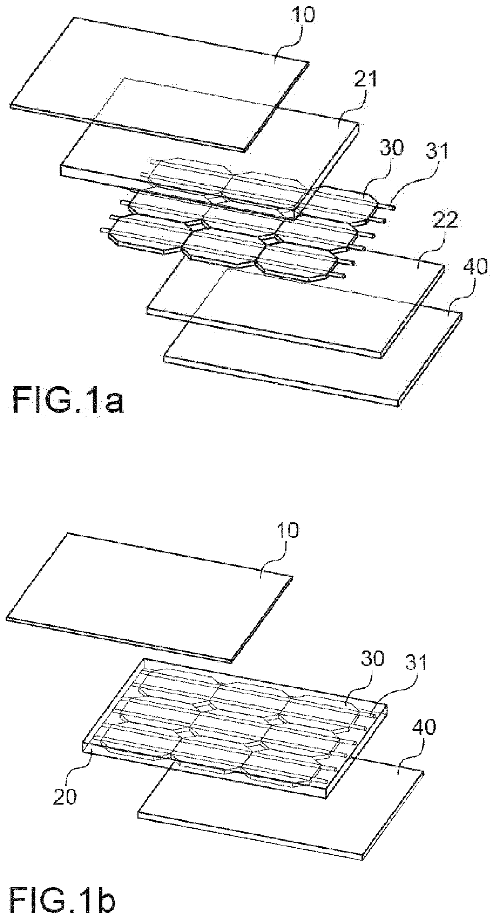

La réticulation a lieu lors de l'étape de lamination. Après l'opération de lamination, les films encapsulants 21, 22 fondent pour ne former qu'une seule couche 20 dite couche d'encapsulation dans laquelle sont noyées les cellules photovoltaïques 30 (figure 1b).

Avantageusement, la résistivité des films encapsulants 21, 22 varie significativement lors de la réticulation.

Par varie significativement, on entend que la résistivité du matériau réticulé est au moins deux fois et, de préférence, au moins dix fois plus élevée que celle du matériau polymère non réticulé. Elle peut, par exemple, être cent fois plus élevée.

Par matériau polymère, on entend un homopolymère ou un copolymère pouvant éventuellement contenir des additifs.

Le matériau polymère est, avantageusement, choisi parmi les homopolymères ou les copolymères d'éthylène. Toute polyoléfine réticulable pouvant être laminée pourrait également être utilisée. De préférence, le matériau polymère est de l'EVA.

Le matériau polymère peut comprendre, en outre, un agent de réticulation, tel qu'un peroxyde, un co-agent ou promoteur de réticulation pour améliorer la cinétique de réaction, un agent de couplage pour améliorer l'adhésion du film sur les plaques avant et arrière ainsi que sur l'ensemble de cellules photovoltaïques interconnectées (par exemple, un silane), un stabilisant UV (molécules capables de piéger des radicaux formés lors du vieillissement UV par recombinaison), et/ou un absorbeur UV tels que les benzophénones, les benzotriazoles, les cyanocrylates et les triazines.

Le premier et le deuxième films encapsulants 21, 22 peuvent être de même nature ou de natures différentes. Au moins le matériau polymère du film encapsulant 21 en face avant est transparent. Par transparent, on entend qu'il laisse passer plus de 70% du rayonnement incident, et de préférence au moins 80%.

Les films encapsulant 21, 22 ont une épaisseur comprise, typiquement, entre 10µm et 5mm, de préférence entre 50µm et 600µm, encore plus préférentiellement entre 200 et 600µm et encore plus préférentiellement entre 400 et 600µm .

Le premier et le deuxième films encapsulants 21, 22 peuvent avoir la même épaisseur ou des épaisseurs différentes.

Les films encapsulants 21, 22 peuvent être maintenus, lors de l'assemblage, sur les cellules photovoltaïques et/ou sur les plaques avant ou arrière grâce à un adhésif sensible à la pression ou à une colle liquide par exemple.

Dans le cadre de la fabrication d'un module photovoltaïque, au moins l'une des plaques qui fera face aux faces actives des cellules photovoltaïques du module est transparente, de sorte à laisser passer le rayonnement électromagnétique. Dans ce mode de réalisation particulier, il s'agit de la première plaque 10, positionnée en face avant. De préférence, la plaque transparente est en verre, ou en polymère, et présente des caractéristiques adaptées à une utilisation dans le domaine photovoltaïque.

La plaque 40 positionnée en face arrière peut être opaque. Il s'agit, par exemple, d'une feuille multicouche, en polymère blanc, et pouvant contenir une feuille en aluminium.

La première plaque 10 et la deuxième plaque 40 ont une résistivité inférieure ou égale, respectivement, à la résistivité du premier film encapsulant laminé 21 et à la résistivité du deuxième film encapsulant laminé 22.

### Assemblage du module photovoltaïque et polymérisation/réticulation de la résine

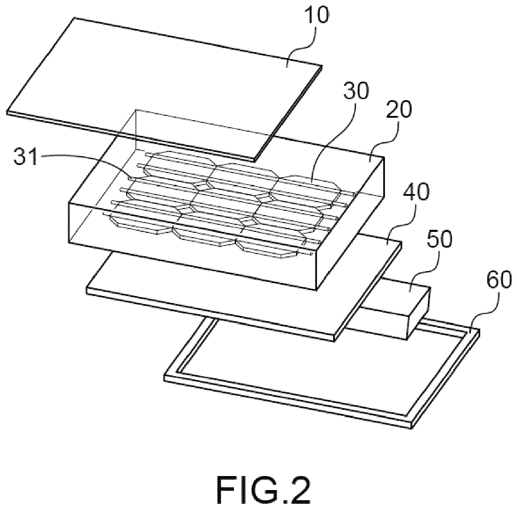

On se réfère à la figure 2 qui représente une vue éclatée d'un module photovoltaïque (PV) selon un mode de réalisation particulier de l'invention. Le module photovoltaïque comprend :
- une première plaque 10, en face avant du module,
- un ensemble de cellules photovoltaïques 30, par exemple, en silicium interconnectées entre elles, par des connections électriquement conductrices,
- une couche d'encapsulation 20 à l'état solide (couche encapsulante), enrobant l'ensemble de cellules photovoltaïques 30,
- une deuxième plaque 40, en face arrière.

La couche d'encapsulation 20 à l'état solide peut être, par exemple, obtenue en venant déposer une formulation visqueuse (pâte), sous forme de « cordons » sur l'une des plaques, puis en la polymérisant (i.e. en la durcissant). Il est également possible d'injecter une formulation à l'état liquide entre les deux plaques puis en la polymérisant.

La formulation initiale contient au moins les précurseurs de la résine. Par précurseur, on entend des monomères et/ou des oligomères et/ou des pré-polymères menant à la formation de la résine polymérique

Avantageusement, on choisira une résine 20 dont la résistivité varie significativement entre l'état dit liquide (résine non polymérisée/réticulée) et l'état dit solide (résine polymérisée/réticulée).

Par varie significativement, on entend que la résistivité à l'état solide est au moins deux fois et, de préférence, au moins dix fois plus élevée que la résistivité de la formulation initiale. Elle peut, par exemple, être cent fois plus élevée.

Selon un premier mode de réalisation avantageux, la résine 20 est une résine durcissable par apport de chaleur.

Selon un autre mode de réalisation avantageux, la résine 20 est une résine photopolymérisable.

Par exemple, la résine est une résine polysiloxane (aussi appelée résine silicone). La résine peut comprendre un mélange de plusieurs polysiloxanes différent. Alternativement, elle peut comprendre un mélange d'un ou plusieurs polysiloxanes avec un ou plusieurs autres polymères (non polysiloxanes). Le composant majoritaire en poids (plus de 50% massique) de la résine est le polysiloxane. Le ou les polysiloxanes de la résine peuvent comprendre des groupements vinyliques et/ou aromatiques tels que des groupes phényle ou benzyle.

De préférence, le polysiloxane est un homopolymère, c'est-à-dire qu'il n'est constitué que d'un seul motif répétitif siloxane. A titre illustratif, il peut s'agir de polydiméthylsiloxane (PDMS)...

La résine polysiloxane peut être obtenue en mélangeant deux formulations, la première contenant un polysiloxilane avec des groupements vinyliques, et la seconde contenant un agent de réticulation siloxane associé à un catalyseur (à base de platine, par exemple). Le mélange des deux formulations déclenche la polymérisation de la résine.

Selon un autre exemple, la résine est une résine acrylique. Par résine acrylique, on entend un homopolymère ou un copolymère acrylique ou méthacrylique. La résine liquide utilisée peut être polymérisée et réticulée, par l'action d'un rayonnement ultraviolet et/ou d'un rayonnement visible.

La résine peut comprendre, ou être constituée, de polyméthacrylate de méthyle (PMMA), de polyméthacrylate de phényle, de polyméthacrylate de benzyle, de polyméthacrylate d'isobornyle, ou d'un copolymère à base de deux ou plus des monomères méthacrylate de méthyle, méthacrylate de phényle, méthacrylate de benzyle, méthacrylate d'isobornyle. Par exemple, il peut également s'agir d'un copolymère à blocs acryliques.

La formulation liquide photopolymérisable utilisée pour réaliser la résine acrylique peut comprendre au moins :
- un ou plusieurs types d'amorceurs photosensibles dont la photolyse génère des espèces réactives qui pourront initier la polymérisation du ou des monomères ;
- un ou plusieurs types de monomères monofonctionnels composés de fonctions réactives et permettant d'ajuster la viscosité du milieu réactionnel ;
- un ou plusieurs types de monomères ou oligomères bi- ou polyfonctionnels, souvent téléchéliques (les fonctions réactives en polymérisation se présentent dans les extrémités des molécules) permettant la réticulation du polymère.

La formulation liquide comprend, par exemple, un mélange de monomères (méth)acryliques, d'oligomères fonctionnalisés avec des groupes (méth)acryliques et des photo-amorceurs.

La formulation liquide peut comporter, en outre, des additifs modifiant la rhéologie de la résine liquide, par exemple des charges organiques ou inorganiques, et/ou des additifs modifiant les propriétés de mouillage de la résine liquide, par exemple des tensioactifs.

La couche d'encapsulation 20 à l'état solide est, avantageusement, transparente. Par transparente, on entend qu'elle laisse passer plus de 70% du rayonnement incident, et de préférence au moins 80%.

La couche d'encapsulation 20 à l'état solide a une épaisseur comprise, typiquement, entre 10µm et 5mm, de préférence entre 50µm et 600µm, encore plus préférentiellement entre 200 et 600µm et encore plus préférentiellement entre 300 et 600µm .

Dans le cadre de la fabrication d'un module photovoltaïque, au moins l'une des plaques qui fera face aux faces actives des cellules photovoltaïques du module est transparente, de sorte à laisser passer le rayonnement électromagnétique. Dans ce mode de réalisation particulier, il s'agit de la première plaque 10, positionnée en face avant. De préférence, la plaque transparente est en verre, ou en polymère, et présente des caractéristiques adaptées à une utilisation dans le domaine photovoltaïque.

Avantageusement, pour la mise en œuvre de modules PV avec des résines liquides à durcir, on utilisera des plaques avant et arrière rigides par exemple en verre.

Afin de réaliser le procédé, la première plaque 10 et la deuxième plaque 40 ont, avantageusement, une résistivité inférieure ou égale à la résistivité de la couche d'encapsulation 20 et de la formulation liquide correspondante.

Pour les deux modes d'assemblage précédemment décrits, les cellules photovoltaïques 30 peuvent être bi-faciales, et les première et deuxième plaques 10, 40 sont transparentes au rayonnement solaire incident.

Les cellules photovoltaïques 30 peuvent être reliées électriquement en série par des connecteurs électriquement conducteurs 31 destinés à collecter l'électricité générée par les cellules photovoltaïques. Les connecteurs électriquement conducteurs 31 sont des connections métalliques, par exemple il s'agit de rubans ou de fils en cuivre. Les cellules sont, avantageusement, régulièrement espacées. Les cellules sont, par exemple, sous la forme de fines plaques d'environ 200µm d'épaisseur. Il y a par exemple 60 cellules dans un module PV. L'ensemble formé par les cellules photovoltaïques 30 et les connecteurs 31 forme un squelette de cellules photovoltaïques.

Le module photovoltaïque peut également comprendre une boite de jonction 50 (ou boite de dérivation) raccordant les connections métalliques 31 du circuit de cellules photovoltaïques 30, et, éventuellement, un cadre en aluminium 60, comme par exemple représenté sur la figure 2.

### Caractérisation du taux moyen de réticulation

Le procédé de caractérisation comprend les étapes successives suivantes :
a) fournir un module photovoltaïque tel que défini précédemment,
b) recouvrir une des plaques 10, 40, par exemple, la plaque 10 en face avant, par un élément électriquement conducteur 100,
c) mesurer la résistance électrique de la couche d'encapsulation 20, avec un appareil de mesure,
d) comparer la résistance électrique mesurée avec des valeurs de référence de modules de même configuration (dimensions, matériaux, etc) de manière à déterminer le taux moyen de réticulation de la couche d'encapsulation 20.

Le procédé comprend, de préférence, entre l'étape b) et l'étape c), les étapes suivantes :
b1) connecter la pluralité de cellules 30 et l'élément électriquement conducteur 100, d'une part, à une source 200 de tension ou de courant, et d'autre part, à un appareil de mesure,
b2) appliquer une tension ou un courant entre la pluralité de cellules 30 et l'élément électriquement conducteur 100.

Le taux moyen de réticulation correspond au taux de réticulation mesuré dans un volume de la couche d'encapsulation 20. Le volume est situé entre les cellules 30 connectées au générateur de tension 200 (et à l'appareil de mesure) et l'élément électriquement conducteur 100. Le taux peut varier localement dans ce volume (dans le cas d'un matériau bien réticulé, on peut observer généralement une variation de moins de 10 %). Il peut varier, par exemple, si la répartition des agents de réticulation est inhomogène dans le film encapsulant 21, 22 ou, dans le cas d'une résine, si la répartition des différents composants de la formulation est inhomogène, ou encore si la température ou l'irradiation n'est pas homogène.

Le taux moyen de réticulation est, dans le cadre de l'invention, déterminé in situ, à partir de la mesure d'une grandeur électrique.

Lorsque l'on mesure le taux moyen de réticulation de la couche d'encapsulation 20 entre la plaque 10, en face avant, et la pluralité de cellules photovoltaïques 300, ceci correspond au taux moyen de réticulation du premier film encapsulant laminé, en face avant.

Les étapes b) à d) peuvent ensuite être répétées, sur l'autre face du module photovoltaïque. Dans ce mode de réalisation, il s'agit de la face arrière. Cela permet de déterminer également le taux moyen de réticulation du film encapsulant laminé, positionné en face arrière ou le taux moyen de réticulation de la couche d'encapsulation 20, obtenue à partir d'une résine liquide, en face arrière. Les taux moyens de réticulation en face avant et en face arrière peuvent être identiques ou différents. Le même élément électriquement conducteur 100 peut être utilisé ou un autre élément électriquement conducteur.

L'élément électriquement conducteur 100 est, par exemple, une feuille d'aluminium ou de cuivre. L'élément électriquement conducteur 100 a une épaisseur allant de 8 à 150µm par exemple. La surface de l'élément électriquement conducteur 100 est avantageusement identique à celle des cellules photovoltaïques 300 connectées au générateur 200 de tension/courant et à l'appareil de mesure. Par exemple, la surface de l'élément électriquement conducteur 100 peut recouvrir la totalité de la surface des cellules 30. L'élément électriquement conducteur 100 est, de préférence, à une distance minimale de 1 mm, préférentiellement de 10 mm à 15 mm, du cadre ou du bord du module PV. Avantageusement, on dispose une charge, par exemple, d'au moins 10Pa, et de préférence d'au moins 30Pa sur l'élément électriquement conducteur 100, de manière à améliorer le contact entre celui-ci et la plaque du module PV.

Alternativement, le cadre du module 60, recouvrant le pourtour de la plaque 10 en face avant pourrait être utilisé comme élément électriquement conducteur pour remplacer la feuille conductrice.

Lors de l'étape c), les cellules PV 300 et l'élément électriquement conducteur 100 sont reliés à la fois à un appareil configuré pour délivrer une grandeur électrique, comme un courant ou une tension, et à un appareil de lecture permettant de déterminer directement ou indirectement la valeur de la résistance de la couche d'encapsulation 20. Par exemple, il s'agit d'un voltmètre ou d'un ampèremètre, ou encore d'un ohmmètre.

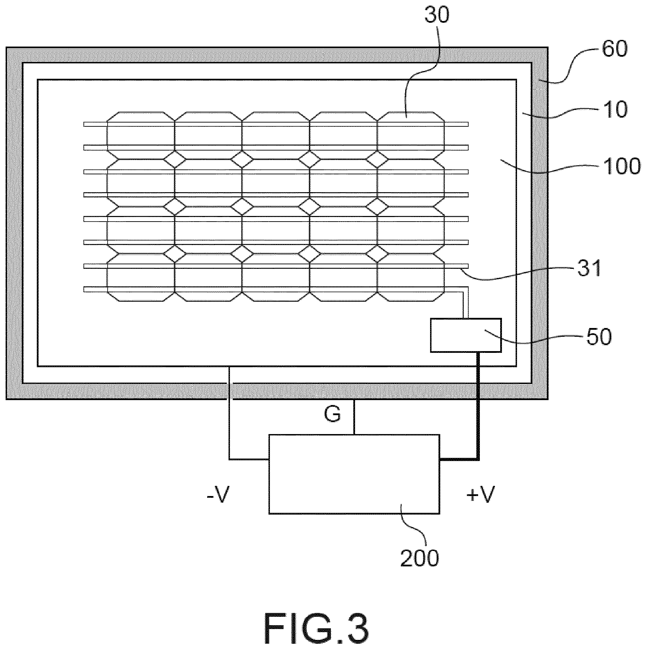

À titre illustratif, la figure 3 représente un montage électrique permettant de mesurer la résistance d'une couche encapsulante 20 d'un module photovoltaïque, selon un mode de réalisation particulier de l'invention.

Les cellules PV 300 sont préférentiellement connectées à la borne positive du générateur 200 de tension/courant. Toutes les cellules ou seulement une partie des cellules peuvent être reliées, par exemple, un tiers des cellules. La borne négative du générateur 200 est préférentiellement reliée à l'élément électriquement conducteur 100. Le générateur 200 est, par exemple, un testeur d'isolement électrique. Dans le cas d'un module PV ayant un cadre 60, le cadre du module 60 est, avantageusement, connecté à la garde du testeur d'isolement pour éviter que le courant aille dans le cadre du module 60. Alternativement, il est possible de recouvrir les bords du module d'une feuille conductrice et de la connecter à la garde du testeur d'isolement.

Lors de l'étape b2), on applique une tension (ou un courant) entre les bornes positive et négative, à travers la couche encapsulante 20. Avantageusement, la tension est d'au moins 250V, par exemple 500V ou 1000V. Dans le cas d'un courant, on choisira un courant de l'ordre du µA. La tension ou le courant sont adaptés en fonction de la surface du module. On applique, avantageusement, la tension ou le courant progressivement. Par exemple, pour une tension, on choisira 500 V/s. Avantageusement, la tension ou le courant est appliqué pendant une durée d'au moins une minute, par exemple deux minutes, avant de mesurer la résistance du film encapsulant laminé, de manière à avoir un régime stable. L'étape b2) peut être répétée plusieurs fois à différentes tensions ou différents courants. Par exemple, elle peut être répétée deux fois. Par exemple, pour un module de taille standard 1,6m² (module de 60 cellules), la tension est répétée une première fois à une tension de 1000V et une seconde fois à une tension de 500V. La tension ou le courant sont adaptés à la surface du module. La répétition de l'étape d) permet d'avoir un procédé plus fiable et d'éviter l'influence des éléments capacitifs du module. Avantageusement, entre chaque valeur de tension appliquée, la tension est coupée, et le module déchargé en court-circuitant les bornes du test.

Lors de l'étape d) la résistance est déterminée à partir d'une grandeur électrique (tension ou courant). Pour cela, un appareil de lecture du courant ou de tension, par exemple avec un voltmètre, peut être utilisé. Il est également possible de déterminer une autre grandeur électrique, comme la résistivité et la conductivité.

La valeur ainsi obtenue est comparée avec des données dites de référence. Les valeurs de référence sont prédéfinies et forment des abaques pour un matériau donné. Les valeurs de référence représentent la résistance, la résistivité ou la conductivité en fonction du taux de réticulation pour un matériau donné. Le taux de réticulation peut être déterminé, préalablement, par analyse calorimétrique différentielle (DSC) par exemple.

On estime que les matériaux polymères laminés sont suffisamment réticulés, pour des applications PV, si le taux moyen de réticulation est supérieur à 60%, et de préférence supérieur à 75%, par exemple pour un taux moyen de réticulation compris entre 60% et 90%, et de préférence compris entre 75% et 85%.

On estime que les matériaux polymères, obtenus à partir d'une résine liquide, sont suffisamment polymérisés/réticulés, pour des applications PV, si le taux moyen réticulation est supérieur à 95%, et de préférence supérieur à 98%.

À la fin du procédé, la tension est coupée, et le module déchargé en court-circuitant les bornes du test.

Avantageusement, le procédé est réalisé à température ambiante (de l'ordre de 20-25°C) et à pression ambiante (de l'ordre de 1bar). On choisira, par exemple, une humidité relative inférieure à 55%. Avantageusement, toutes les mesures sont réalisées dans les mêmes conditions environnementales.

En fonction des valeurs obtenues, il est également possible de modifier les paramètres de la chaine de fabrication pour la fabrication des modules photovoltaïques suivants.

En particulier, il est possible de modifier les paramètres liés à l'étape de réticulation des matériaux destinés à former la couche d'encapsulation.

Dans le cas d'une couche d'encapsulation 20 obtenue par laminage de film(s) encapuslant(s), il est, par exemple, possible d'augmenter la température ou la durée de l'étape de lamination. La fabrication des modules est ainsi optimisée.

Dans le cas d'une couche d'encapsulation 20 obtenue à partir d'une résine liquide, si le taux moyen de réticulation ainsi déterminé est inférieur à une valeur seuil prédéterminée, il est possible de soumettre le module PV à une nouvelle étape de polymérisation, sous irradiation UV ou par un apport de chaleur par exemple. La nouvelle étape de polymérisation peut être réalisée avec les mêmes paramètres ou avec des paramètres différents. Il est, par exemple, possible d'augmenter la température, la puissance de l'irradiation ou la durée de l'étape de polymérisation. Le taux moyen de réticulation de la couche d'encapsulation 20 peut ensuite être de nouveau déterminé. Ces étapes (détermination du taux de réticulation puis polymérisation) peuvent être répétées jusqu'à obtenir un taux de réticulation supérieur à la valeur seuil prédéfinie.

Avec un tel procédé, il est également possible de suivre sur le site de production l'avancement de la polymérisation/réticulation de la couche d'encapsulation 20, par exemple, en fonction du temps suite à la lamination du module, et donc la qualité du module photovoltaïque au cours de sa fabrication ou avant sa sortie du site de production.

Il est également possible de récupérer des modules PV ayant déjà servi, et présentant des défaillances, afin de vérifier le taux moyen de réticulation de la couche d'encapsulation. Ceci permet de réaliser un diagnostic et de vérifier si la défaillance provient des couches d'encapsulation des modules PV ou d'un autre élément. Ceci peut être utile, par exemple, si l'on constate que les plaques en face avant et arrière présentent un décalage ou dans le cas de réactions dues à des précurseurs résiduels.

Exemples illustratifs et non limitatifs d'un mode de réalisation :

### Exemple 1-

### Détermination du taux moyen de réticulation d'échantillons laminés dans des conditions industrielles :

Différents modules contenant de l'EVA, à 33 % d'acétate de vinyle, ont été assemblés. Les modules comprennent un empilement comprenant successivement :
- une plaque 10 en verre (3 mm d'épaisseur),
- une feuille d'encapsulant 21 d'EVA de 450µm d'épaisseur,
- un squelette de cellules interconnectées 30,
- une deuxième feuille d'encapsulant 22 d'EVA de 450µm d'épaisseur,
- une plaque 40 (« backsheet ») de type TPT de 330 µm d'épaisseur.

Les modules sont ensuite laminés à des temps différents dans un laminateur industriel de la société 3S, modèle S1815E. Le laminateur est un laminateur à deux chambres, une chambre supérieure et une chambre inférieure, séparées par une membrane souple. L'empilement est placé dans la chambre inférieure, chauffée par le bas par un plateau chaud. Après une première phase de dégazage pendant laquelle du vide est appliqué aux deux chambres, la chambre supérieure est remise à pression atmosphérique, ce qui a pour effet de venir plaquer la membrane souple qui sépare les deux chambres, contre l'empilement.

Pour obtenir un module ayant des films non réticulés (taux moyen de réticulation de 0%, ou proche de 0%), le module est laminé à basse température, par exemple selon le cycle de lamination suivant :
- 4 à 5 min de dégazage avec le module en contact avec la plaque chauffant,
   7 à 8 min sous 1000mbar,
   température de la plaque chauffante du laminateur à 100°C.

Pour obtenir un module ayant des films très réticulés (taux moyen de réticulation de 80%), le module est laminé avec un traitement thermique plus important en termes de température et de durée, selon par exemple le cycle de lamination suivant :
- 4 à 5 min de dégazage avec le module en contact avec la plaque chauffante,
- 30 min de réticulation sous 1000mbar,
- température de la plaque chauffante du laminateur à 160°C.

La mesure de résistance électrique des différents modules est réalisée, en face avant du module, à température de 21,7°C et à une humidité relative 43%, selon le procédé suivant :
- connecter les deux bornes du module en court-circuit à la borne positive d'un testeur d'isolement électrique,
- appliquer une feuille électriquement conductrice 100 (une feuille d'aluminium ou de cuivre de 8 µm à 150 µm) sur la face avant du module, de manière recouvrir à 100% la surface des cellules 300, la feuille électriquement conductrice 100 étant distante du cadre 60 du module d'au moins 1 mm, préférentiellement de 10 mm à 15 mm,
- appliquer une charge homogène, de préférence d'au moins 30 Pa, sur la surface conductrice,
- connecter cette feuille conductrice 100 à la borne négative du testeur 200 d'isolement électrique,
- connecter le cadre 60 du module à la garde du testeur 200 d'isolement, -appliquer progressivement (à 500 V/s) une tension de 3000V pendant 2 minutes puis mesurer la résistance électrique,
- une fois la tension coupée, décharger le module en court-circuitant les bornes du test puis enlever le court-circuit.

Un morceau de feuille d'EVA réticulée est ensuite récupéré sur le module PV et caractérisée par DSC.

La courbe de résistance d'isolation du module en fonction du taux de réticulation mesuré peut ensuite être tracée. A partir de cette courbe de référence, il est possible de déterminer le taux moyen de réticulation d'un film d'encapsulation en EVA laminé d'un module photovoltaïque in situ et sans détériorer le module PV.

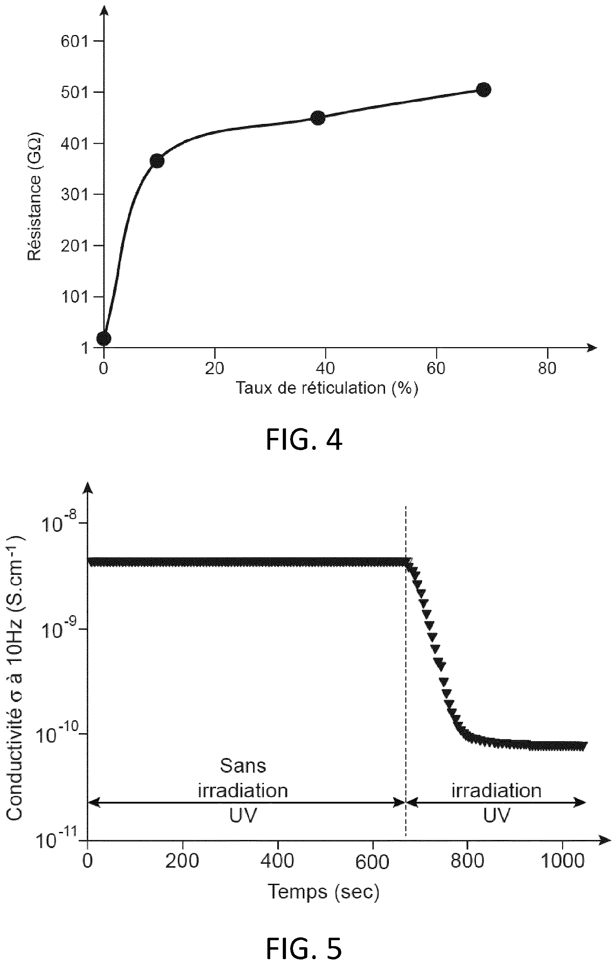

La figure 4 représente la résistance de l'EVA en fonction du taux moyen de réticulation. Chaque paramètre a été mesuré trois fois. La valeur représente la moyenne de 3 mesures. La variation de résistance électrique de l'EVA permet d'obtenir la courbe de référence et de déterminer par la suite le taux moyen de réticulation d'un film d'encapsulation en EVA laminé d'un module photovoltaïque in situ et sans détériorer le module PV.

### Exemple 2-

### Evolution de la conductivité d'un échantillon de résine acrylique en fonction du temps, avec et sans irradiation UV :

La figure 5 représente la réponse diélectrique d'une résine acrylique. Les mesures ont été réalisées à 10Hz, pour avoir une réponse de type résistif. La conductivité de la résine polymérisée diminue de deux ordres de grandeur par rapport à la résine non polymérisée (formulation liquide).

### Comparaison du taux de conversion déterminé par mesure diélectrique et taux de conversion mesuré par spectroscopie Raman

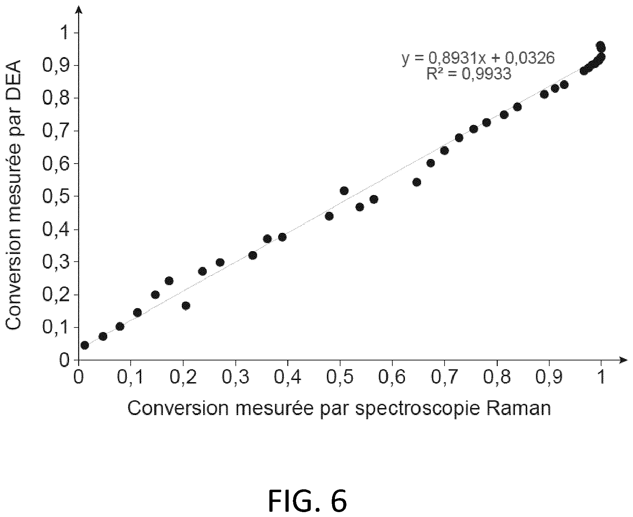

Les conversions mesurées par spectroscopie diélectrique et celles mesurées par spectroscopie Raman ont été tracées l'une en fonction de l'autre (figure 6), d'après l'article de Hardis et al. précédemment décrit *("*Cure kinetics characterization and monitoring of an epoxy resin using DSC, Raman spectroscopy, and DEA", Compos. Part Appl. Sci. Manuf. 49 (2013) 100-108). Les conversions évaluées par des analyses diélectriques suivent la même tendance que celles observées par spectroscopie Raman confirmant que la mesure de la résistivité est une méthode fiable pour déterminer le taux moyen de conversion d'une résine. La relation est linéaire. A 90% de conversion chimique, la conductivité, traduisant la mobilité des ions, atteint son minimum.

### Détermination du taux de conversion d'échantillons polymérisés dans des conditions industrielles

Différents modules PV contenant une couche d'encapsulation 20 obtenue à partir d'une résine acrylique ont été assemblés. Les modules comprennent un empilement comprenant successivement :
- une plaque 10 en verre (3mm d'épaisseur)
- une couche de résine 20 silicone de 450µm d'épaisseur finale,
- un squelette de cellules interconnectées 30,
- une autre plaque de verre 40.

Pour obtenir une résine encapsulante non polymérisée/réticulée (non convertie), le module n'est pas soumis à une étape d'irradiation UV.

Pour obtenir des modules avec des résines polymérisées/réticulées (converties), les modules sont soumis à différentes durées d'irradiation sous rayonnement UV, sous une puissance de 0,5W/cm². Par exemple, il est possible de réaliser cinq durées d'irradiation différentes.

La mesure de résistance électrique des différents modules est réalisée, en face avant du module, à température de 21,7 °C et à une humidité relative 43%, selon le procédé suivant :
- connecter les deux bornes du module en court-circuit à la borne positive d'un testeur d'isolement électrique,
- appliquer une feuille conductrice 100 (une feuille d'aluminium ou de cuivre de 8 µm à 150 µm) sur la face avant du module, de manière recouvrir à 100% la surface des cellules 30, la feuille conductrice 100 étant distante du cadre du module d'au moins 1 mm, préférentiellement de 10mm à 15mm,
- appliquer une charge homogène, de préférence d'au moins 30 Pa, sur la feuille conductrice 100,
- connecter cette feuille conductrice 100 à la borne négative du testeur d'isolement électrique,
- connecter le cadre du module à la garde du testeur d'isolement,
- appliquer progressivement (à 500 V/s) une tension de 3000V pendant 2 minutes puis mesurer la résistance électrique,
- une fois la tension coupée, décharger le module en court-circuitant les bornes du test puis enlever le court-circuit.

Le module PV est ensuite désassemblé et la couche de résine 20 est récupérée. Le taux moyen de conversion est obtenu en caractérisant la résine par DSC et en déterminant sa température de transition vitreuse.

À partir de ces données, la courbe représentant la résistance de la couche de résine en fonction de son taux moyen de conversion peut être tracée. A partir de cette courbe de référence, il est possible de déterminer le taux moyen de conversion d'une couche de résine d'un module photovoltaïque in situ et sans détériorer le module PV.

## Revendications

1. Procédé de caractérisation du taux moyen de réticulation d'une couche d'encapsulation (20), présente dans un module photovoltaïque, comprenant les étapes successives suivantes :
a) fournir un module photovoltaïque comprenant au moins :
- une première plaque (10),
- une pluralité de cellules photovoltaïques (30), reliées électriquement entre elles,
- une couche d'encapsulation (20), encapsulant la pluralité de cellules photovoltaïques (30),
- une deuxième plaque (40), la couche d'encapsulation (20) et la pluralité de cellules photovoltaïques (30) étant situées entre les première et deuxième plaques (10, 40),
b) recouvrir la première plaque (10) par un élément électriquement conducteur (100),
c) mesurer la résistance électrique de la couche d'encapsulation (20) entre la pluralité de cellules photovoltaïques (30) et l'élément électriquement conducteur (100), avec un appareil de mesure,
d) comparer la résistance électrique mesurée avec des valeurs de référence prédéfinies, de manière à déterminer le taux moyen de réticulation de la couche d'encapsulation (20).

2. Procédé selon la revendication 1, **caractérisé en ce que** le procédé comporte, en outre, après l'étape d), les étapes successives suivantes :
e) retirer l'élément électriquement conducteur (100) de la première plaque (10) et recouvrir la deuxième plaque (40) avec l'élément électriquement conducteur (100),
f) répéter étapes c) et d) telles que définies dans la revendication 1.

3. Procédé selon l'une des revendications précédentes, **caractérisé en ce que** le procédé comporte entre l'étape b) et l'étape c) les étapes suivantes :
b1) connecter la pluralité de cellules photovoltaïques (30) et l'élément électriquement conducteur (100), d'une part, à une source (200) de tension ou de courant, et d'autre part, à l'appareil de mesure,
b2) appliquer une tension ou un courant entre la pluralité de cellules photovoltaïques (30) et l'élément électriquement conducteur (100).

4. Procédé selon la revendication précédente, **caractérisé en ce que** la tension appliquée lors de l'étape b2) est supérieure ou égale à 250V, par exemple 500V ou 1000V.

5. Procédé selon l'une des revendications 3 et 4, **caractérisé en ce que** le courant ou la tension appliquée lors de l'étape b2) est appliquée pendant une durée d'au moins 30 secondes, et de préférence d'au moins 1 minute.

6. Procédé selon l'une des revendications 3 à 5, **caractérisé en ce que** l'étape b2) est répétée deux fois, par exemple une première fois à une tension de 1000V et une deuxième fois à une tension de 500V.

7. Procédé selon l'une quelconque des revendications précédentes, **caractérisé en ce qu'**une pression d'au moins 10Pa est appliquée sur l'élément électriquement conducteur (100), lors des étapes b2) et c).

8. Procédé selon l'une quelconque des revendications précédentes, **caractérisé en ce que** le procédé comporte entre l'étape c) et l'étape d) les étapes suivantes :
c1) débrancher l'appareil de mesure, et
c2) décharger le module photovoltaïque en court-circuitant des parties électriquement conductrices du module photovoltaïque avec la terre.

9. Procédé selon l'une quelconque des revendications 1 à 8, **caractérisé en ce que** la couche d'encapsulation (20) est en copolymère d'éthylène-acétate de vinyle, ou en un autre copolymère à base d'éthylène.

10. Procédé selon l'une quelconque des revendications 1 à 8, **caractérisé en ce que** la couche d'encapsulation (20) est une couche de résine acrylique.

11. Procédé selon l'une quelconque des revendications 1 à 8, **caractérisé en ce que** la couche d'encapsulation (20) est une couche de résine silicone.

12. Procédé selon l'une quelconque des revendications 1 à 11, **caractérisé en ce que** l'élément électriquement conducteur (100) recouvre la pluralité de cellules photovoltaïques (30).

13. Procédé selon l'une quelconque des revendications précédente, **caractérisé en ce que** l'élément électriquement conducteur (100) est une feuille métallique, par exemple une feuille d'aluminium ou de cuivre.

14. Procédé selon l'une quelconque des revendications 1 à 11, **caractérisé en ce que** l'élément électriquement conducteur (100) est un cadre (60) disposé sur le pourtour du module photovoltaïque.

15. Procédé de suivi qualité d'une ligne de fabrication de modules photovoltaïques, comprenant les étapes successives suivantes :
i. assembler au moins :
- une première plaque (10),
- un premier film d'encapsulation réticulable (21),
- une pluralité de cellules photovoltaïques (30), reliées électriquement entre elles,
- un deuxième film d'encapsulation réticulable (22),
- une deuxième plaque (40).
ii. laminer l'ensemble obtenu à l'étape i, de manière à faire fondre et au moins partiellement réticuler le premier film d'encapsulation réticulable (21) et le deuxième film d'encapsulation réticulable (22), moyennant quoi on forme un module photovoltaïque ayant une couche d'encapsulation (20) encapsulant la pluralité de cellules photovoltaïques (20),
iii. réaliser le procédé de caractérisation du taux moyen de réticulation de la couche d'encapsulation (20), telles que définies dans l'une quelconque des revendications 1 à 14, pour suivre la qualité du module photovoltaïque.

16. Procédé de suivi qualité d'une ligne de fabrication de modules photovoltaïques, comprenant les étapes successives suivantes :
i'. assembler au moins :
- une première plaque (10),
- une couche de formulation liquide polymérisable/réticulable,
- une pluralité de cellules photovoltaïques (30),
- une deuxième plaque (40),
ii'. Faire polymériser/réticuler, la formulation liquide polymérisable/réticulable, moyennant quoi on forme un module photovoltaïque ayant une couche d'encapsulation (20) encapsulant la pluralité de cellules photovoltaïques (20),
iii'. Réaliser le procédé de caractérisation du taux moyen de réticulation de la couche d'encapsulation (20), telles que définies dans l'une quelconque des revendications 1 à 14, pour suivre la qualité du module photovoltaïque.

17. Procédé de suivi qualité d'une ligne de fabrication de modules photovoltaïques selon l'une des revendications 15 ou 16, **caractérisé en ce que** si le taux moyen de réticulation de la couche d'encapsulation (20) est inférieur à une valeur seuil prédéfinie, au moins un des paramètres de la ligne de fabrication du module photovoltaïque, par exemple, choisi parmi la température, la pression, la durée de l'étape de lamination et la durée de l'étape de polymérisation/réticulation, est modifié.

## Patentansprüche

1. Verfahren zur Charakterisierung der durchschnittlichen Vernetzungsrate einer Einkapselungsschicht (20), die in einem photovoltaischen Modul vorhanden ist, umfassend die nachstehenden, aufeinanderfolgenden Schritte:
a) Bereitstellen eines photovoltaischen Moduls, das zumindest enthält:
- eine erste Platte (10),
- eine Vielzahl von photovoltaischen Zellen (30), die elektrisch miteinander verbunden sind,
- eine Einkapselungsschicht (20), die die Vielzahl von photovoltaischen Zellen (30) einkapselt,
- eine zweite Platte (40), wobei sich die Einkapselungsschicht (20) und die Vielzahl von photovoltaischen Zellen (30) zwischen der ersten und der zweiten Platte (10, 40) befinden,
b) Abdecken der ersten Platte (10) mit einem elektrisch leitenden Element (100),
c) Messen des elektrischen Widerstands der Einkapselungsschicht (20) zwischen der Vielzahl von photovoltaischen Zellen (30) und dem elektrisch leitenden Element (100) mit einer Messeinrichtung,
d) Vergleichen des gemessenen elektrischen Widerstands mit vordefinierten Referenzwerten, um die durchschnittliche Vernetzungsrate der Einkapselungsschicht (20) zu bestimmen.

2. Verfahren nach Anspruch 1, **dadurch gekennzeichnet, dass** das Verfahren ferner nach Schritt d) die nachstehenden, aufeinanderfolgenden Schritte umfasst:
e) Entfernen des elektrisch leitenden Elements (100) von der ersten Platte (10) und Abdecken der zweiten Platte (40) mit dem elektrisch leitenden Element (100),
f) Wiederholen der Schritte c) und d) wie in Anspruch 1 definiert.

3. Verfahren nach einem der vorhergehenden Ansprüche, **dadurch gekennzeichnet, dass** das Verfahren zwischen Schritt b) und Schritt c) die nachstehenden Schritte umfasst:
b1) Verbinden der Vielzahl von photovoltaischen Zellen (30) und des elektrisch leitenden Elements (100) einerseits mit einer Spannungs- oder Stromquelle (200) und andererseits mit der Messeinrichtung,
b2) Anlegen einer Spannung oder eines Stroms zwischen der Vielzahl von photovoltaischen Zellen (30) und dem elektrisch leitenden Element (100).

4. Verfahren nach dem vorhergehenden Anspruch, **dadurch gekennzeichnet, dass** die in Schritt b2) angelegte Spannung größer oder gleich 250 V, beispielsweise 500 V oder 1000 V, ist.

5. Verfahren nach einem der Ansprüche 3 und 4, **dadurch gekennzeichnet, dass** der in Schritt b2) angelegte Strom oder die angelegte Spannung für eine Dauer von zumindest 30 Sekunden, vorzugsweise von zumindest 1 Minute, angelegt wird.

6. Verfahren nach einem der Ansprüche 3 bis 5, **dadurch gekennzeichnet, dass** Schritt b2) zweimal wiederholt wird, beispielsweise ein erstes Mal bei einer Spannung von 1000 V und ein zweites Mal bei einer Spannung von 500 V.

7. Verfahren nach einem der vorhergehenden Ansprüche, **dadurch gekennzeichnet, dass** in den Schritten b2) und c) ein Druck von zumindest 10 Pa auf das elektrisch leitende Element (100) aufgebracht wird.

8. Verfahren nach einem der vorhergehenden Ansprüche, **dadurch gekennzeichnet, dass** das Verfahren zwischen Schritt c) und Schritt d) die nachstehenden Schritte umfasst:
c1) Abschalten der Messeinrichtung, und
c2) Entladen des photovoltaischen Moduls durch Kurzschließen elektrisch leitender Teile des photovoltaischen Moduls gegen Erde.

9. Verfahren nach einem der Ansprüche 1 bis 8, **dadurch gekennzeichnet, dass** die Einkapselungsschicht (20) aus Ethylen-Vinylacetat-Copolymer oder einem anderen Copolymer auf Ethylenbasis besteht.

10. Verfahren nach einem der Ansprüche 1 bis 8, **dadurch gekennzeichnet, dass** die Einkapselungsschicht (20) eine Acrylharzschicht ist.

11. Verfahren nach einem der Ansprüche 1 bis 8, **dadurch gekennzeichnet, dass** die Einkapselungsschicht (20) eine Silikonharzschicht ist.

12. Verfahren nach einem der Ansprüche 1 bis 11, **dadurch gekennzeichnet, dass** das elektrisch leitende Element (100) die Vielzahl von photovoltaischen Zellen (30) abdeckt.

13. Verfahren nach einem der vorhergehenden Ansprüche, **dadurch gekennzeichnet, dass** das elektrisch leitende Element (100) eine Metallfolie, beispielsweise eine Aluminium- oder Kupferfolie, ist.

14. Verfahren nach einem der Ansprüche 1 bis 11, **dadurch gekennzeichnet, dass** das elektrisch leitende Element (100) ein am Umfang des photovoltaischen Moduls angeordneter Rahmen (60) ist.

15. Verfahren zur Qualitätskontrolle einer Fertigungslinie für photovoltaische Module, umfassend die nachstehenden, aufeinanderfolgenden Schritte:
i. Zusammenfügen von zumindest:
- einer ersten Platte (10),
- einer ersten vernetzbaren Einkapselungsfolie (21),
- einer Vielzahl von photovoltaischen Zellen (30), die elektrisch miteinander verbunden sind,
- einer zweiten vernetzbaren Einkapselungsfolie (22),
- einer zweiten Platte (40),
ii. Laminieren der in Schritt i. erhaltenen Anordnung, um die erste vernetzbare Einkapselungsfolie (21) und die zweite vernetzbare Einkapselungsfolie (22) zu schmelzen und zumindest teilweise zu vernetzen, wodurch ein photovoltaisches Modul mit einer Einkapselungsschicht (20) gebildet wird, die die Vielzahl von photovoltaischen Zellen (20) einkapselt,
iii. Durchführen des Verfahrens zur Charakterisierung der durchschnittlichen Vernetzungsrate der Einkapselungsschicht (20), wie in einem der Ansprüche 1 bis 14 definiert, zur Qualitätskontrolle des photovoltaischen Moduls.

16. Verfahren zur Qualitätskontrolle einer Fertigungslinie für photovoltaische Module, umfassend die nachstehenden, aufeinanderfolgenden Schritte:
i'. Zusammenfügen von zumindest:
- einer ersten Platte (10),
- einer Schicht aus polymerisierbarer/vernetzbarer flüssiger Formulierung,
- einer Vielzahl von photovoltaischen Zellen (30),
- einer zweiten Platte (40),
ii'. Polymerisieren/Vernetzen der polymerisierbaren/vernetzbaren flüssigen Formulierung, wodurch ein photovoltaisches Modul mit einer Einkapselungsschicht (20) gebildet wird, die die Vielzahl von photovoltaischen Zellen (20) einkapselt,
iii'. Durchführen des Verfahrens zur Charakterisierung der durchschnittlichen Vernetzungsrate der Einkapselungsschicht (20), wie in einem der Ansprüche 1 bis 14 definiert, zur Qualitätskontrolle des photovoltaischen Moduls.

17. Verfahren zur Qualitätskontrolle einer Fertigungslinie für photovoltaische Module nach einem der Ansprüche 15 oder 16, **dadurch gekennzeichnet, dass** dann, wenn die durchschnittliche Vernetzungsrate der Einkapselungsschicht (20) niedriger als ein vordefinierter Schwellenwert ist, zumindest einer der Parameter der Fertigungslinie für photovoltaische Module, beispielsweise ausgewählt aus Temperatur, Druck, Dauer des Laminierungsschritts und Dauer des Polymerisations-/Vernetzungsschritts, verändert wird.

## Claims

1. A method for characterising the average crosslinking rate of an encapsulation layer (20), present in a photovoltaic module, comprising the following successive steps of:
a) providing a photovoltaic module comprising at least:
- a first plate (10),
- a plurality of photovoltaic cells (30), electrically connected to one another,
- an encapsulation layer (20), encapsulating the plurality of photovoltaic cells (30),
- a second plate (40), the encapsulation layer (20) and the plurality of photovoltaic cells (30) being located between the first and second plates (10, 40),
b) covering the first plate (10) by an electrically conductive element (100),
c) measuring the electrical resistance of the encapsulation layer (20) between the plurality of photovoltaic cells (30) and the electrically conductive element (100), with a measurement apparatus,
d) comparing the electrical resistance measured with predefined reference values, so as to determine the average crosslinking rate of the encapsulation layer (20).

2. The method according to claim 1, **characterised in that** the method further includes, after step d), the following successive steps of:
e) removing the electrically conductive element (100) from the first plate (10) and covering the second plate (40) with the electrically conductive element (100),
f) repeating steps c) and d) such as defined in claim 1.

3. The method according to one of the preceding claims, **characterised in that** the method includes between step b) and step c) the following steps of:
b1) connecting the plurality of photovoltaic cells (30) and the electrically conductive element (100), on the one hand, to a voltage or current source (200), and on the other hand, to the measurement apparatus,
b2) applying a voltage or a current between the plurality of photovoltaic cells (30) and the electrically conductive element (100).

4. The method according to the preceding claim, **characterised in that** the voltage applied during step b2) is greater than or equal to 250V, for example 500V or 1 000V.

5. The method according to one of claims 3 to 4, **characterised in that** the current or the voltage applied during step b2) is applied for a time length of at least 30 seconds, and preferably at least 1 minute.

6. The method according to one of claims 3 to 5, **characterised in that** step b2) is repeated twice, for example a first time with a voltage of 1 000V and a second time with a voltage of 500V.

7. The method according to any of the preceding claims, **characterised in that** a pressure of at least 10Pa is applied to the electrically conductive element (100), during steps b2) and c).

8. The method according to any of the preceding claims, **characterised in that** the method includes between step c) and step d) the following steps of:
c1) disconnecting the measurement apparatus, and
c2) discharging the photovoltaic module by short-circuiting electrically conductive parts of the photovoltaic module with the earth.

9. The method according to any of claims 1 to 8, **characterised in that** the encapsulation layer (20) is made of ethylene-vinyl acetate copolymer, or of another ethylene based copolymer.

10. The method according to any of claims 1 to 8, **characterised in that** the encapsulation layer (20) is an acrylic resin layer.

11. The method according to any of claims 1 to 8, **characterised in that** the encapsulation layer (20) is a silicone resin layer.

12. The method according to any of claims 1 to 11, **characterised in that** the electrically conductive element (100) covers the plurality of photovoltaic cells (30).

13. The method according to any of the preceding claims, **characterised in that** the electrically conductive element (100) is a metal sheet, for example an aluminum or copper sheet.

14. The method according to any of claims 1 to 11, **characterised in that** the electrically conductive element (100) is a frame (60) disposed on the periphery of the photovoltaic module.

15. A method for monitoring the quality of a photovoltaic module manufacturing line, comprising the following successive steps of:
i. assembling at least:
- a first plate (10),
- a first crosslinkable encapsulation film (21),
- a plurality of photovoltaic cells (30), electrically connected to one another,
- a second crosslinkable encapsulation film (22),
- a second plate (40).
ii. laminating the assembly obtained at step i, so as to melt and at least partially crosslink the first crosslinkable encapsulation film (21) and the second crosslinkable encapsulation film (22), whereby a photovoltaic module having an encapsulation layer (20) encapsulating the plurality of photovoltaic cells (20) is formed,
iii. performing the method for characterising the average crosslinking rate of the encapsulation layer (20), such as defined in any of claims 1 to 14, for monitoring the quality of the photovoltaic module.

16. A method for monitoring the quality of a photovoltaic module manufacturing line, comprising the following successive steps of:
i.' assembling at least:
- a first plate (10),
- a polymerisable/crosslinkable liquid formulation layer,
- a plurality of photovoltaic cells (30),
- a second plate (40),
ii'. polymerising/crosslinking, the polymerisable/crosslinkable liquid formulation, whereby a photovoltaic module having an encapsulation layer (20) encapsulating the plurality of photovoltaic cells (20) is formed,
iii'. performing the method for characterising the average crosslinking rate of the encapsulation layer (20), such as defined in any of claims 1 to 14, for monitoring the quality of the photovoltaic module.

17. The method for monitoring the quality of a photovoltaic module manufacturing line according to one of claims 15 or 16, **characterised in that** if the average crosslinking rate of the encapsulation layer (20) is lower than a predefined threshold value, at least one of the parameters of the photovoltaic module manufacturing line, for example, selected from temperature, pressure, time length of the laminating step and time length of the polymerising/crosslinking step, is modified.
